(19)

**Europäisches Patentamt**
**European Patent Office**
**Office européen des brevets**

(11) **EP 3 502 727 B1**

(12) **EUROPEAN PATENT SPECIFICATION**

(45) Date of publication and mention
of the grant of the patent:
**26.01.2022 Bulletin 2022/04**

(21) Application number: **17209923.6**

(22) Date of filing: **22.12.2017**

(51) International Patent Classification (IPC):
**G01R 33/56** *(2006.01)*     **G01R 33/54** *(2006.01)*
**A61B 5/055** *(2006.01)*

(52) Cooperative Patent Classification (CPC):
**G01R 33/5608; A61B 5/0037; A61B 5/055;**
**A61B 5/7203; G01R 33/543**

(54) **METHOD FOR DETERMINING AN IMAGING QUALITY INFORMATION FOR A MAGNETIC RESONANCE IMAGING APPARATUS FROM A SIGNAL-TO-NOISE RATIO AT AN ANATOMICAL LANDMARK**

VERFAHREN ZUR BESTIMMUNG EINER ABBILDUNGSQUALITÄTSINFORMATION FÜR EINE MAGNETRESONANZBILDGEBUNGSVORRICHTUNG AUS EINEM SIGNAL-ZU-RAUSCH VERHÄLTNIS BEI EINEM ANATOMISCHEN MERKPUNKT

PROCÉDÉ PERMETTANT DE DÉTERMINER UNE INFORMATION DE QUALITÉ D'IMAGERIE POUR UN APPAREIL D'IMAGERIE PAR RÉSONANCE MAGNÉTIQUE D'UN RAPPORT SIGNAL-BRUIT À UN REPÈRE ANATOMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.06.2019 Bulletin 2019/26**

(73) Proprietor: **Siemens Healthcare GmbH**
**91052 Erlangen (DE)**

(72) Inventors:
• **de Oliveira, Andre**
**91080 Uttenreuth (DE)**
• **Harder, Martin**
**90429 Nürnberg (DE)**
• **Kannengießer, Stephan**
**42113 Wuppertal (DE)**
• **Landschütz, Wilfried**
**91083 Baiersdorf (DE)**

(56) References cited:
**US-A1- 2012 010 495     US-A1- 2015 199 478**
**US-A1- 2017 156 630**

• **MAXIMILIAN DE BUCOURT ET AL: "Obese patients in an open MRI at 1.0 Tesla: image quality, diagnostic impact and feasibility", EUROPEAN RADIOLOGY, SPRINGER, BERLIN, DE, vol. 21, no. 5, 3 December 2010 (2010-12-03), pages 1004-1015, XP019894751, ISSN: 1432-1084, DOI: 10.1007/S00330-010-2005-2**
• **"MS1-2008 Determination of signal-to-noise ratio (SNR) in diagnostic magnetic resonance images", NEMA STANDARDS PUBLICAT, NATIONAL MANUFACTURERS ELECTRICAL ASSOCIATION, no. MS 1-2008, 1 January 2008 (2008-01-01), pages 1-19, XP007915348,**
• **Robert W. Brown ET AL: "15.3 Contrast, Contrast-to-Noise, and Visibility" In: "Magnetic Resonance Imaging : Physical Principles and Sequence Design, Second Edition", 14 April 2014 (2014-04-14), Wiley, XP055484520, ISBN: 978-0-471-72085-0 pages 342-343, * section "15.3 Contrast, Contrast-to-Noise, and Visibility" ***

**EP 3 502 727 B1**

Printed by Jouve, 75001 PARIS (FR)

**Description**

The invention relates to a method for determining an imaging quality information for a magnetic resonance imaging apparatus in an examination process of a patient positioned at least partly within an imaging volume of the magnetic resonance imaging apparatus, wherein the imaging quality information is used for assessing the performance of the magnetic resonance imaging apparatus and/or for determining imaging parameters for an imaging protocol.

A magnetic resonance imaging apparatus is a complex system composed of hundreds of subcomponents which are very sensitive to environmental changes. Therefore, the magnetic resonance imaging apparatus must be constantly monitored to guarantee patient and staff safety as well as a high image quality. Typically, the components of a magnetic resonance imaging apparatus must be tested periodically during quality assurance measurements to check whether the components are working within specified ranges. These quality assurance measurements require phantom measurements to ensure that the results of the experiments are reproducible and comparable to previous measurements. However, during these phantom measurements, the magnetic resonance imaging apparatus is not available for patient examination. It is therefore desirable to reduce the number of such quality assurance measurements based on phantom measurements to increase the capacity utilisation of the magnetic resonance imaging apparatus.

US 2012/0010495 A1 describes the generation of magnetic resonance images of a volume section within an examination object by way of a magnetic resonance scanner, in which a number of quality inspections is performed on at least one magnetic resonance image. In the case of failed inspections, an action is automatically performed in order to improve a quality when generating more of the magnetic resonance images. The automatically performed quality inspection shortens a wait time of a patient in the magnetic resonance imaging apparatus. Based on the result of the inspection of the at least one magnetic resonance image, an action can be performed in order to improve the quality of magnetic resonance images generated after this action.

MAXIMILIAN DE BUCOURT ET AL.: "Obese patients in an open MRI at 1.0 Tesla: image quality, diagnostic impact and feasibility",EUROPEAN RADIOLOGY, SPRINGER, BERLIN, DE, vol. 21, no. 5, 3 December 2010, discloses a method for determining an image quality information for an open magnetic resonance imaging apparatus in obese patients.

US 2017/156630 A1 discloses a method for determining an image quality information for a magnetic resonance imaging apparatus, wherein the imaging protocol is optimized based on a patient tissue attribute.

However, it would be desirable to not only improve the upcoming images of an examination procedure, but also to gain information about the performance of the magnetic resonance imaging apparatus in order to eliminate or reduce the number of service staff visits needed to ensure that the system is operating according to its specification.

Therefore, the object of the invention is to provide a method to obtain quality information during the examination of different patients, wherein the quality information is not entirely patient-specific and therefore comparable over several examinations.

According to the invention, this object is achieved by a method for determining an imaging quality information for a magnetic resonance imaging apparatus in an examination process of a patient positioned at least partly within an imaging volume of the magnetic resonance imaging apparatus, wherein the imaging quality information is used for assessing the performance of the magnetic resonance imaging apparatus and/or for determining imaging parameters for an imaging protocol, comprising the following steps:

- measuring a noise information describing the general noise properties of the magnetic resonance imaging apparatus in the imaging volume using a predetermined first magnetic resonance sequence,
- acquiring patient image data in at least one imaging region of the patient using a predetermined second magnetic resonance sequence, wherein a magnetic resonance sequence which is independent of the individual patient, meaning that no parameters of the sequence are adapted to the patient, is used as the second magnetic resonance sequence,
- localizing at least one anatomical landmark of a group of predetermined anatomical landmarks in the patient image by a landmark detection algorithm, wherein for each landmark and/or for at least one pair of landmarks, a reference information regarding the imaging quality information is provided in a database,
- determining a landmark-specific signal-to-noise ratio for each localized landmark from the patient image data at the landmark and the noise information, and
- determining the imaging quality information from the landmark-specific signal-to-noise ratio, wherein the assessment of the performance of the magnetic resonance imaging apparatus and/ or the determination of the imaging parameters is performed depending on a comparison of the imaging quality information and the corresponding reference information in the database.

The invention is based on the insight that landmark-specific signal-to-noise ratios can be used to reduce individual variations of patient-specific measurements since a certain type of landmarks comprises a certain type of tissue, which has a defined range of relevant magnetic resonance imaging parameters like T1, T2, proton density, or other parameters. Using the same magnetic resonance sequence, the landmark will thus have similar signal intensities for different patients. Therefore, this tissue-specific or landmark-specific signal-to-noise ratio for a predetermined group of landmarks can be used as a reproducible measurement for the evaluation of the performance of the magnetic resonance imaging apparatus if it is determined using the same magnetic resonance imaging sequences for all patients. Additionally or alternatively, these landmark-specific signal-to-noise ratios, as a measure of imaging quality, may enable an adaption or an optimization of imaging protocols.

The determination of the landmark-specific signal-to-noise ratio may, in particular, be performed at least once for each patient, for example at the beginning of the examination process of the patient with the magnetic resonance imaging apparatus, in particular as an integral part of the examination process, obviating the need for dedicated, separate quality assurance measurements. Since the fluctuation of tissue composition at the landmark between different patients is sufficiently low, at least for the group of landmarks for which a reference information has been stored in the database, comparison of values for different examinations and thus different patients is in particular possible, as will be further described below. In particular, the reference information may comprise or be derived from imaging quality information or comparison results of previous examinations. Thus, in embodiments, the reference information in the database may be updated depending on the current imaging quality information and/or comparison results.

It is one advantage of the method according the invention, that, based on the usage of landmark-specific signal-to-noise ratios, imaging quality information obtained for different patients is comparable and therefore permits the assessment of the magnetic resonance imaging apparatus performance, so that phantom measurements for quality assurance measurement can be eliminated or reduced in number.

A second advantageous aspect of the method according to the invention is the usage of these landmark-specific signal-to-noise ratios for the determination of imaging parameters, which could be used, for example, for an adaption or for an optimization of an imaging protocol. For instance, the imaging parameters can be determined for and used in sequences and/or protocols, which will be conducted subsequently in the ongoing examination process. For example, a database of imaging parameters associated with certain ranges of landmark-specific signal-to-noise ratios can be provided. However, the invention mainly aims at quality assessment of the magnetic resonance imaging apparatus.

It is noted that, of course, it is not required for the patient image to show every landmark for which a reference information is obtainable from the database. In many cases, only parts of the patient will be inside the imaging volume of the magnetic resonance apparatus, such that, preferably, the database contains reference information for a group of landmarks essentially uniformly distributed over the body. In an embodiment, the landmark detection algorithm may detect available landmarks and check if these landmarks are also present in the database. For the detected at least one landmark for which a reference information is available, the landmark-specific signal-to-noise ratio is determined.

The determination of a signal-to-noise ratio requires at least two different values, as both an information about the signal and an information about the noise is needed. The information about the noise is determined by measuring a noise information, which describes the general noise properties of the magnetic resonance imaging apparatus in the imaging volume. For that, the predetermined first magnetic resonance sequence is used to perform a noise measurement by recording a noise level present in the at least one coil element used for measuring, in particular one or more RX channels of a body coil. Such a noise measurement can be performed for instance every time that a new coil configuration is selected or that a table of the magnetic resonance imaging apparatus, on which the patient is positioned, is moved.

The second value for the signal-to-noise ratio, namely the signal information, is determined by acquiring patient image data in the at least one imaging region of the patient using a predetermined second magnetic resonance sequence. Using the second magnetic resonance sequence, the patient image data in at least one imaging region of the patient is measured. In this patient image data, at least one anatomical landmark of a patient or a pair of anatomical landmarks of the patient can be localized by a landmark detection algorithm. At least one anatomical landmark of a group of predetermined anatomical landmarks available for the imaging region can be used. For each anatomical landmark or for each pair of anatomical landmarks, which is available for localization, a reference information regarding the imaging quality information is provided in a database. The database can be part of the magnetic resonance imaging apparatus or it can be an external database, which can be connected to the magnetic resonance imaging apparatus for instance by a communication network, so that the database can be accessed by the magnetic resonance imaging apparatus or so that the magnetic resonance imaging apparatus can request information from the database.

For the extraction of the landmark from the patient image data, different methods and/or algorithms are known in the state of the art. For example, a landmark localization algorithm described by Zhou et al. (X. S. Zhou, Z. Peng, Y. Zhan, M. Dewan, B. Jian, A. Krishnan, Y. Tao, M. Harder, S. Gross-kopf, and U. Feuerlein. Redundancy, redundancy, redundancy: the three keys to highly robust anatomical parsing in medical images. In MIR '10: Proc. Int'l Conf. Multimedia Info Retrieval, pages 175-184, New York, NY, USA, 2010) can be used in a method according to the invention. When the at least one anatomical landmark or pair of anatomical landmarks is localized within the patient image data, a signal value describing the signal level at the landmark or at each landmark of a pair of landmarks can be extracted from the patient image data. From this signal level information, the landmark-specific signal-to-noise ratio for each localized landmark can be determined by , in particular, dividing by the noise information.

It shall be noted that it is insignificant whether the noise information is determined before the patient image data is acquired or if the steps are conducted vice versa. Hence, the steps of measuring the noise information and of acquiring the patient image data can be conducted in an arbitrary order.

The landmark-specific signal-to-noise ratio is used for the determination of the imaging quality information. As a consequence, also the imaging quality information is landmark-specific. To assess the performance of the magnetic resonance imaging apparatus and/or for a determination of the imaging parameters, the imaging quality information is compared to the corresponding reference information regarding the imaging quality information in the database. This comparison between the currently determined imaging quality information and the stored reference information enables the assessment of the performance of the magnetic resonance imaging apparatus and/or the determination of the imaging parameters.

The usage of a landmark-specific signal-to-noise ratio allows a comparison of patient image data collected for different patients, since properties of the landmark, like for instance signal level from T1 decays, T2 decays, T2* decays, proton densities and others, as well as standard deviations for these values measured for different patients are known. For instance, the standard variation of T1 or T2 decays, respectively, lies within the range of 20% to 30% for measurements on different patients. This standard deviation is less than the standard deviation which would be obtained by measuring the signal-to-noise ratio at an arbitrary position within the patient image data depicting an arbitrary type of tissue. Furthermore, for a measurement in an arbitrary position, no reference information enabling the assessment of the performance of the magnetic resonance imaging apparatus and/or the determination of the imaging parameters can be provided. Criteria for selecting landmarks for which reference values are to be provided in the database may comprise a standard deviation of measured magnetic resonance signals for a representative population of patients to be below a certain threshold and/or criteria relating to their detectability in patient image data acquired using the predetermined second magnetic resonance sequence.

According to the invention, a magnetic resonance sequence which is independent of the individual patient, in particular a pre-scan sequence or a localizer sequence, is used as the second magnetic resonance sequence. By the usage of a sequence which is conducted independently on the patient, which means that no parameters of the sequence are adapted to the patient, the comparability of different landmark-specific signal-to-noise ratios can be enabled since the patient image data is obtained using an equal or a similar sequence. It can be advantageous to use, for instance, a pre-scan sequence or a localizer sequence, which is conducted at the beginning of every examination process with identical or nearly identical parameters. In this manner, the determination of the imaging quality information is integrated into the examination process without the need to add further measurements regarding the signal information. In this respect, a noise measurement may also already be performed for other purposes. Preferably, the measurement sequence protocol of the second magnetic resonance sequence is kept constant or close to constant for different patient examination processes.

Preferably, a magnetic resonance sequence without excitation pulses is used as first magnetic resonance sequence. The noise information can be measured using this first magnetic resonance sequence without excitation pulses and/or by performing a read-out with no gradients applied. By using a sequence without excitation, no magnetic resonance signal is created and only the noise level, which can comprise, for instance, stochastic noise, and/or noise arising from the setup, is measured.

In a preferred embodiment of the invention, it may be provided that the first magnetic sequence corresponds to the second magnetic resonance sequence without excitation pulses. For example, the noise measurement is conducted using the first magnetic resonance sequence which is the same as the second magnetic resonance sequence except for the excitation pulses. In particular, all parameters of the first magnetic resonance sequence, which are not related to the generation of excitation pulses, can be equal or similar to the parameters of the second magnetic resonance sequence. In this way, the noise information is obtained by a first magnetic resonance sequence which is similar to the second magnetic resonance sequence used for the measuring of the patient image data.

Embodiments of the invention may provide that a reference information comprising a reference signal-to-noise ratio is used, wherein the reference signal-to-noise ratio is calculated and/or empirically determined and/or determined based on previously measured landmark-specific signal-to-noise ratios stored in the database, in particular as a mean or a weighted average of previously measured landmark-specific signal-to-noise-ratios. For the assessment of the performance of the magnetic resonance imaging apparatus and/or for the determination of the imaging parameters, the imaging quality information determined from the landmark-specific signal-to-noise ratio of the current examination process is compared to the statistically and/or empirically and/or theoretically determined reference information from the database.

The reference information can comprise, for instance, a reference signal-to-noise ratio, which can be calculated, for example, using simulation algorithms, theoretical frameworks and/or the like, or it can be empirically determined from a number of previously conducted calibration measurements, for example directly after the magnetic resonance apparatus has been installed. It is also possible that the reference information comprises landmark-specific signal-to-noise ratios previously measured in actual examination processes and/or at least one mean or weighted average of such previously measured landmark-specific signal-to-noise ratios.

An embodiment of the method according to the invention may provide that the landmark-specific signal-to-noise ratio is determined by averaging a signal intensity within a defined image region comprising or comprised by the landmark. For example, for each landmark, a rectangular or quadratic image region, which comprises the landmark, can be used for the determination of the signal intensity. For example, the signal intensity within a defined number of pixels or voxels of the patient image data comprising the landmark can be averaged by calculating for instance the mean of the signal intensity of each pixel or voxel of the image region and by using the mean as the signal intensity of the landmark for the determination of the landmark-specific signal-to-noise ratio.

Embodiments of the invention may provide that the imaging quality information comprises an average of the current landmark-specific signal-to-noise ratio and previously measured landmark-specific signal-to-noise ratios stored in the database, in particular as a mean and/or a sliding-window average and/or a weighted average of the previously measured landmark-specific signal-to-noise ratios. For example, a defined number of previously measured landmark-specific signal-to-noise ratios as well as the signal-to-noise ratio of the current examination process can be used to determine an average of the landmark-specific signal-to-noise ratios as imaging quality information to allow a more robust estimation. It is possible to use a weighted average, which weights recently acquired signal-to-noise ratios higher than older signal-to-noise ratios.

The average may also be a sliding-window average based on a defined number of previously measured landmark-specific signal-to-noise ratios and the current landmark-specific signal-to-noise ratio. The sliding window average may be determined, for instance, from the current landmark-specific signal-to-noise ratio and nine previously determined landmark-specific signal-to-noise ratios from the database, which were determined during the nine preceding examination processes. Of course, also a weighting of the previously measured signal-to-noise ratio can be conducted while determining the sliding-window average, so that for instance recently determined signal-to-noise ratios are weighted higher than older ones.

The determined imaging quality information is compared to the reference information to assess the performance of the magnetic resonance imaging apparatus. Preferably, the comparison may comprise the determination of a trend information, which describes the behaviour of the imaging quality information, in particular landmark-specific signal-to-noise ratios, over all or a defined number of previously performed measurements. For that purpose, an imaging quality information comprising the currently determined landmark-specific signal-to-noise ratio and a reference information comprising previously determined landmark-specific signal-to-noise ratios can be compared. A decreasing landmark-specific signal-to-noise ratio can be an indicator that a calibration of the magnetic resonance imaging apparatus should be performed. To reduce the influence of measurement errors or statistical fluctuations, it is also possible that statistical averages are used, for example an imaging quality information comprising an average of the currently determined landmark-specific signal-to-noise ratio and a defined number previously determined landmark-specific signal-to-noise ratios and corresponding averages for earlier time intervals as reference information. For instance, an average of the current and nine preceding signal-to-noise ratios can be compared to the reference information, which comprises for instance a threshold signal-to-noise ratio or an average of fifty previously determined signal-to-noise ratios for the specific landmark, so that a decrease and/or a deterioration of the landmark-specific signal-to-noise ratio can be determined enabling, for instance, the assessment of the performance of the magnetic resonance imaging apparatus.

It may be provided in embodiments of the invention that a notification to an operator of the magnetic resonance imaging apparatus is generated if the performance of the magnetic resonance imaging apparatus assessed from the comparison of the imaging quality information and the reference information meets at least one notification criterion. As notification criterion, for instance, a minimally allowed landmark-specific signal-to-noise ratio can be used as a threshold. It is also possible that a certain decrease rate in signal-to-noise ratios within a defined number of previously performed measurements is used as a notification criterion, which enables the detection of a decreasing or deteriorating performance before the landmark-specific signal-to-noise ratio drops below the threshold. In particular, a prediction of when the threshold is reached can be determined depending on trend information. A notification to an operator of the magnetic resonance imaging apparatus may be generated by notifying her or him on a signal-to-noise ratio below the threshold or on an ongoing decrease of the signal-to-noise ratios within a defined number of last measurements or within a defined time period or a soon to occur drop of the signal-to-noise ratio below the threshold or the like.

In a preferred embodiment of the invention, it may be provided that a contrast-to-noise ratio for a pair of landmarks is determined as imaging quality information, wherein the reference information comprises a reference contrast-to-noise ratio. The contrast-to-noise ratio can be calculated based on the determined landmark-specific signal-to-noise ratios for the pair of landmarks. Preferably, two landmarks of different tissue type/composition are used for the determination of the contrast-to-noise ratio. For the assessment of the performance of the magnetic imaging apparatus and/or the determination of the imaging parameters, the imaging quality information comprising a contrast-to-noise ratio can be compared to a reference information which also comprises a reference contrast-to-noise ratio. Thus, in the imaging quality information, the contrast-to-noise ratio can be used as a surrogate for the signal-to-noise ratio itself or additionally to them, so that all remarks above also apply.

In a preferred addition to the inventive method, which may also be used independently of assessing the magnetic resonance imaging apparatus, an optimization of the imaging parameters of at least one imaging sequence of the current examination process may be conducted based on the contrast-to-noise ratio of at least one pair of landmarks. Based on the determined contrast-to-noise ratio from the landmark-specific signal-to-noise ratios of a pair of landmarks, an optimization of the imaging sequence and hence the current examination process can be performed by determining imaging parameters for the imaging sequence of the current examination process. The optimization target is preferably a maximization of the contrast-to-noise ratio between two different landmarks, which facilitates distinguishing for example the tissue of the first landmark from the tissue of second landmark of the pair of landmarks.

Additionally, it may be provided that, for the optimization of the imaging parameters, at least one modified imaging sequence differing from the imaging sequence in at least one imaging parameter value is used, wherein an optimization for the at least one imaging parameter value is conducted based on a comparison of the contrast-to-noise ratio of the imaging sequence and a contrast-to-noise ratio determined from the at least one modified imaging sequence. By using the modified imaging sequence, which differs from the imaging sequence in at least one imaging parameter, also a contrast-to-noise ratio for the modified imaging sequence can be determined from landmark-specific signal-to-noise ratios of a pair of landmarks, so that from a comparison of the contrast-to-noise ratios of the imaging sequence and the modified sequence an optimization of the imaging parameters with respect to the contrast-to-noise ratio can be performed. In particular, the optimization of the imaging parameters can target a maximization of the contrast-to-noise ratio.

It may be provided in an embodiment of the invention that an optimized sequence is generated by multiple usage of differently modified imaging sequences and an optimization of the contrast-to-noise ratio in dependence of the at least one imaging parameter. This allows performing a step-by-step optimization procedure, which is based on the evaluation of the contrast-to-noise ratios for the different modified sequences comprising at least one different imaging parameter. By doing so, the influence of the parameter on the contrast-to-noise ratio can be determined and an optimized parameter value for each imaging parameter can be found leading to an optimized sequence with an optimized or optimal contrast-to-noise ratio. An optimized or optimal contrast-to-noise ratio can be, for example, a high contrast-to-noise ratio between a pair of landmarks.

A magnetic resonance imaging apparatus according to the invention provides that it comprises a control unit, configured to perform a method according to the invention. All remarks regarding the method according to the invention also apply to the magnetic resonance imaging apparatus. The control unit can comprise for instance a noise measurement unit configured to measure the noise information by using the first magnetic resonance sequence. The control unit can also comprise a patient data acquisition unit, configured to acquire the patient image data using the second magnetic resonance sequence. Furthermore, the control unit can comprise a landmark localization unit configured to localize at least one anatomical landmark in the patient image data as well as a signal-to-noise ratio determination and comparison unit, which is configured to determine the landmark-specific signal-to-noise ratio as well as the imaging quality information based on the landmark-specific signal-to-noise ratio, and performing a comparison to the reference information. The reference information can be stored in a database of the magnetic resonance imaging apparatus or in an external database, wherein the control unit is configured to communicate with the database.

A computer program according to the invention comprises instructions which, when the program is executed by a computer, cause the computer to carry out a method according to the invention. The computer is the control unit of the magnetic resonance imaging apparatus or an external computing unit, which communicates with the magnetic resonance imaging apparatus.

An electronically readable storage medium according to the invention has a computer program according to the invention stored thereon.

Additional advantages and features of the invention become evident from the embodiments discussed below as well as from the figures. The figures show:

Fig. 1    a schematic diagram of a method according to the invention, and

Fig. 2    a schematic view of a magnetic resonance imaging apparatus according to the invention.

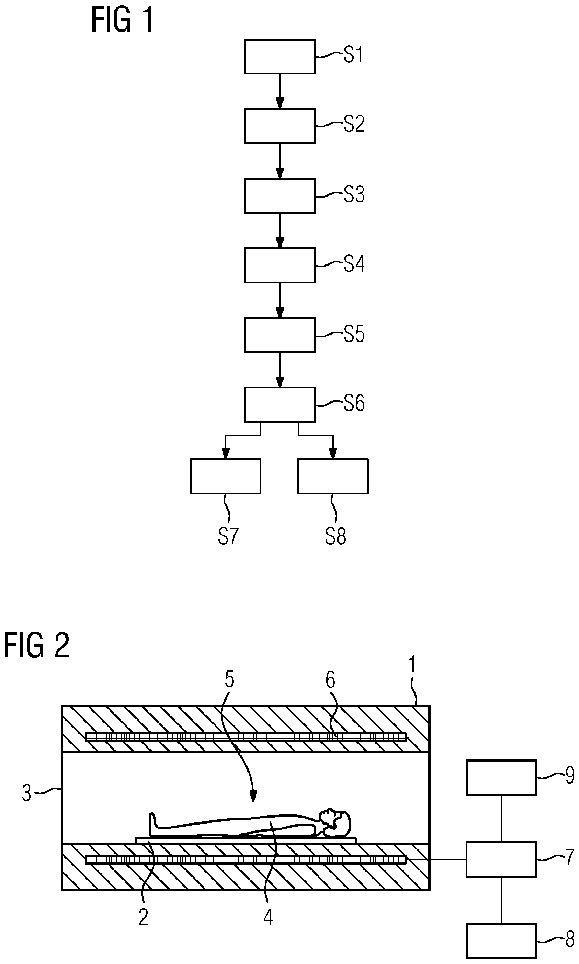

In fig. 1, a flow diagram of a method according to the invention is shown. The steps are numbered as follows:

| S1 | Start |
|----|-------|
| S2 | Measuring the noise information using the first magnetic resonance sequence |
| S3 | Acquisition of patient image data using the second magnetic resonance sequence |
| S4 | Landmark localization |

(continued)

| S5 | Determination of the signal-to-noise ratio |
|----|---------------------------------------------|
| S6 | Determination of the imaging quality information and comparison with the reference information |
| S7 | Assessment of performance and notification |
| S8 | Determination of imaging parameters and optimization of imaging sequence |

The method according to the invention begins in step S1. Preferably, the method is conducted at the beginning of an examination process of a patient. The patient is therefore positioned at least partly within an imaging volume of the magnetic resonance imaging apparatus.

In step S2, a noise information describing the general noise properties of the magnetic resonance apparatus in the imaging volume is measured using a predetermined first magnetic resonance sequence. This first magnetic resonance sequence does not contain any excitation pulses and is used only for measuring or recording of the noise level of at least one coil element used for imaging. As coil elements, for instance, the coil elements of a body coil or another radio-frequency coil of the magnetic resonance imaging apparatus can be used. It is also possible that additional coil elements are used or that a coil element or a plurality of coil elements not including the body coil is used, for example coil elements of, in particular integrated, local coils of the magnetic resonance imaging apparatus. The noise level of the at least one coil element may be determined every time that a new coil configuration is selected for imaging or that the table, on which the patient is positioned, is moved. Based on the results of the noise measurement, for each channel or for each coil, respectively, a complex vector $N_{CHA}$ of length k is obtained as:

$$n_{CHA} = (a_0 + b_0 i; \ a_1 + b_1 i; \dots ; a_k + b_k i),$$

wherein a and b denote each of the k complex measurement values. For each channel or for each coil, the noise level can be calculated as:

$$noise\_level_{CHA} = \frac{\sum_{n=1}^{k} a_n^2 + b_n^2}{k} \quad .$$

This noise level depends on one or more protocol parameters like coil gain and bandwidth, which can be additionally considered when using this information, for instance by the usage of at least one corresponding correction factor. A general noise level denoted as *noise_level* can be obtained by combining the noise levels of all relevant n channels (each corresponding to a coil element) as:

$$noise\_level = [noise\_level_{CHA1} + noise\_level_{CHA2} +, \dots, noise\_level_{CHAn}],$$

 wherein a combined noise vector denoted *combined noise vector* can be calculated as:

$$combined\_noise\_vector \ = noise\_level * noise\_decorr\_matrix,$$

wherein *noise_decorr_matrix* denotes a noise decorrelation matrix. A scalar combined noise level denoted *combined_noise_level* can be calculated as:

$$combined\_noise\_level = $$
$$\sqrt{noise\_level_{CHA1}^2 + noise\_level_{CHA2}^2 +, \dots, noise\_level_{CHAn}^2} \ ,$$

wherein the combined noise level describes the general noise properties of the magnetic resonance apparatus in the imaging volume when using the at least one coil element. The combined noise vector and/or the combined noise level can be used as noise information. Of course it is also possible that another type of noise information and/or another way of calculating a combined noise vector or a combined noise level can be used within the scope of the invention. A combined noise level describes the average noise level within the imaging volume of the magnetic resonance imaging apparatus.

In step S3 of the method, patient image data is acquired in at least one imaging region of the patient using a predetermined second magnetic resonance sequence. As the second magnetic resonance sequence, a pre-scan sequence or a localizer sequence executed in the beginning of the examination process can be used. The patient image data contains at least one patient image of the imaging region of the patient. It is advantageous to use a first magnetic resonance sequence in step S2 resembling the second magnetic resonance sequence except excitation pulses. In this case, the noise information can be determined with equal or close to equal parameters to the acquisition of the patient image data in step S3 except for the excitation. The order of step S2 and S3 is arbitrary; it is therefore possible that the patient image data is acquired before the noise measurement is performed.

In step S4, at least one anatomical landmark and/or at least one pair of anatomical landmarks of a group of predetermined anatomical landmarks is localized in the patient image by a landmark detection algorithm. The calculation of the algorithm can be performed by a control unit of the magnetic resonance imaging apparatus or by a landmark localizing unit of a control unit of the magnetic resonance imaging apparatus. For each of the predetermined anatomical landmarks, a reference information is provided in a database. The database can be part of the magnetic resonance imaging apparatus or it can be an external database which is established to communicate with a control unit of the magnetic resonance imaging apparatus.

In step S5, from the patient image data, a signal intensity of the landmark is obtained. The signal intensity can be calculated for instance for an image area comprising or comprised by the landmark. Assuming for example a punctate landmark, the signal intensity can be calculated for instance as an average of the signal intensities in pixels or voxels around the pixel or voxel of the punctate landmark. For example, a rectangular area of n by m pixels or voxels around the punctual landmark can be used to calculate the signal-to-noise ratio of the i-th landmark *SNR_landmark[i]* as:

$$SNR\_landmark[i] = \frac{\sum_{x=posx\_landmark[i]-n}^{posx\_landmark[i]+n} \sum_{y=posy\_landmark[i]+m}^{posx\_landmark[i]-m} Image(x,y)}{combined\_noise\_level},$$

wherein the nominator denotes the signal intensity of each pixel or voxel within the rectangular n-times m image area *Image(x,y)* of the patient image and wherein the denominator contains the combined noise level as noise information to obtain the signal-to-noise ratio.

In step S6, an imaging quality information is determined from the landmark-specific signal-to-noise ratio, wherein the imaging quality information is compared to the corresponding reference information for the respective landmark, which is stored in the database. It is possible that the landmark-specific signal-to-noise ratio is used as imaging quality information and that it is compared to a reference information, which also comprises a reference signal-to-noise ratio, for instance as a threshold or as a minimally allowed signal-to-noise ratio, which should be obtained by the imaging procedure. It is also possible that the imaging quality information is based on the landmark-specific signal-to-noise ratio determined in step S5 as well as on previously measured and determined landmark-specific signal-to-noise ratios of the respective landmark that have been stored in the database. For instance, the imaging quality information can contain an average of the currently determined and several precedingly determined landmark-specific-to-noise ratios. This average may be a weighted average and/or a sliding-window average that only refers to the currently determined signal-to-noise ratio and a certain number of previously determined signal-to-noise ratios. For instance, it is possible to evaluate the landmark-specific signal-to-noise ratios obtained during the current examination as well as during the last nine examination processes for the respective landmark performed with the magnetic resonance imaging apparatus. Additionally, also a weighting of the different signal-to-noise ratios is possible, so that, for example, the more recently acquired landmark-specific signal-to-noise ratios are weighted higher than the older landmark-specific signal-to-noise ratios. Of course it is also possible to evaluate any other number of signal-to-noise ratios and/or to use another method of weighting.

The reference information may comprise a threshold signal-to-noise ratio, which describes a minimally allowed landmark-specific signal-to-noise ratio. The imaging quality information comprising the currently determined signal-to-noise ratio and/or an average of the currently determined signal-to-noise ratio and several previously determined signal-to-noise ratios can be compared to this reference information to determine whether the currently determined signal-to-noise ratio is above the threshold signal-to-noise ratio.

It is also possible that the reference information comprises several previously determined landmark-specific signal-to-noise ratios or averages thereof, wherein a trend information describing a trend of the currently determined signal-to-noise ratio or an average containing it and a predefined number of previously recorded landmark-specific signal-to-noise ratios or averages thereof is determined by a comparison of the imaging quality information and the reference information. The reference information may comprise a mean or a weighted average of the previously measured landmark-specific signal-to-noise ratios.

Depending on the comparison between the imaging quality information and the reference information in step S6, an assessment of a performance of the magnetic resonance imaging apparatus in step S7 and/or an optimization of imaging parameters in step S8 can be performed.

For the assessment of the performance of the magnetic resonance imaging apparatus in step S7, it is possible to notify an operator of the magnetic resonance imaging apparatus if the comparison between imaging quality information and reference information meets a notification criterion. The performance may be assessed by evaluating whether the currently determined landmark specific signal-to-noise ratio (or an average containing it) is below a threshold reference signal-to-noise ratio. If this is the case, a notification on the necessity of a calibration of the magnetic resonance imaging apparatus can be generated and used to inform an operator of the magnetic resonance imaging apparatus. The notification may be output as a text on a screen of the magnetic resonance imaging apparatus and/or as an audible signal. Such a notification replaces in an advantageous way the necessity for regular periodic calibration procedures, as a notification to an operator occurs when an insufficient signal-to-noise ratio is obtained.

An assessment of the performance is also possible by evaluating a trend within the landmark-specific signal-to-noise ratios during the comparison of the imaging quality information and the reference information. For this purpose, an imaging quality information comprising the currently measured landmark-specific signal-to-noise ratio and a reference information comprising a plurality of previously measured signal-to-noise ratios can be used. From the currently measured landmark-specific signal-to-noise ratio or an average containing it and a number of previously measured signal-to-noise ratios or at least one average thereof, a trend information can be determined, so that for instance a decrease or a deterioration of the landmark-specific signal-to-noise ratio during the last examination procedures can be detected. Additionally or alternatively, as described,, a reference information comprising at least one mean or weighted average of the previously measured landmark-specific signal-to-noise ratios can be used for the determination of the trend information.

Additionally or alternatively to the assessment of the performance of the magnetic resonance imaging apparatus, in step S8 also a determination of imaging parameters for an imaging sequence of the current examination process is possible.

As the noise information has already been determined and the determination of a current landmark-specific signal-to-noise ratio has been established, these informations and steps may be further advantageously exploited. Based on obtained landmark-specific signal-to-noise ratios for a pair of landmarks, an adaption of the imaging parameters of an imaging sequence to be used in the examination process can be performed. This may comprise an optimization of imaging parameters.

To achieve this, a contrast-to-noise ratio CNR(A,B) of a pair of landmarks comprising landmark A and landmark B can be determined as:

$$CNR(A,B) = SNR(A) - SNR(B),$$

wherein SNR (A) and SNR (B) denote the landmark-specific signal-to-noise ratio of landmark A and landmark B, respectively, using the imaging sequence whose imaging parameters are to be optimised. Based on this contrast noise ratio, an optimization of the at least one imaging sequence of the current examination process can be performed by adapting imaging parameters.

In the optimization process, at least one imaging parameter of the imaging sequence may be varied to maximize the contrast to noise ration. Measurement of the contrast-to-noise ration can, in this process, be repeated with a modified imaging sequence modified according to the variation of the image parameters to be adapted. This allows comparison of the previously determined contrast-to-noise ratio with the current contrast to noise ratio resulting from the variation of the imaging parameters, thus assessing the variation. If a termination criterion is then fulfilled, optimization can be terminated, or a new variation of the parameters may be determined and used depending on the optimization algorithm used.

In fig. 2, a magnetic resonance imaging apparatus 1 according to the invention is shown. On a table 2 inside a bore 3 of the magnetic resonance imaging apparatus 1, a patient 4 is positioned. The patient 4 is positioned in an imaging volume 5 of the magnetic resonance imaging apparatus 1. As a coil having at least one coil element for the noise measurement using the first magnetic resonance sequence as well as for the acquisition of the patient image data using the second magnetic resonance sequence, a body coil 6 of the magnetic resonance imaging apparatus 1 can be used. The magnetic resonance imaging apparatus 1 can further comprise a control unit 7, which is configured to perform a method according to the invention. The control unit 7 may comprise a noise measurement unit configured to measure the noise information by using the first magnetic resonance sequence. The control unit 7 may also comprise a patient data acquisition unit, configured to acquire the patient image data using the second magnetic resonance sequence. Furthermore, the control unit 7 may comprise a landmark localization unit configured to localize at least one anatomical landmark in the patient image data as well as a signal-to-noise ratio determination and comparison unit, which is configured to determine the landmark-specific signal-to-noise ratio as well as the imaging quality information based on the landmark-specific signal-to-noise ratio and compare it to a reference info stored in a database 8 of the magnetic resonance imaging apparatus 1. The magnetic resonance imaging apparatus 1 can also comprise a notification means 9 to display a notification in a visual or acoustic manner to an operator of the magnetic resonance imaging apparatus 1, like a loudspeaker or a screen.

Although the present invention has been described in detail with reference to the preferred embodiment, the present invention is not limited by the disclosed examples from which the skilled person is able to derive other variations without departing from the scope of the invention, which is defined by the appended set of claims.

**Claims**

1.  Method for determining an imaging quality information for a magnetic resonance imaging apparatus (1) in an examination process of a patient (4) positioned at least partly within an imaging volume (5) of the magnetic resonance imaging apparatus, wherein the imaging quality information is used for assessing the performance of the magnetic resonance imaging apparatus and/or for determining imaging parameters for an imaging protocol, comprising the following steps:

    - measuring a noise information describing the general noise properties of the magnetic resonance imaging apparatus (1) in the imaging volume (5) using a predetermined first magnetic resonance sequence,
    - acquiring patient image data in at least one imaging region of the patient (4) using a predetermined second magnetic resonance sequence, wherein a magnetic resonance sequence which is independent of the individual patient, meaning that no parameters of the sequence are adapted to the patient, is used as the second magnetic resonance sequence,
    - localizing at least one anatomical landmark of a group of predetermined anatomical landmarks in the patient image by a landmark detection algorithm, wherein for each landmark and/or for at least one pair of landmarks, a reference information regarding the imaging quality information is provided in a database (8),
    - determining a landmark-specific signal-to-noise ratio for each localized landmark from the patient image data at the landmark and the noise information, and
    - determining the imaging quality information from the landmark-specific signal-to-noise ratio, wherein the assessment of the performance of the magnetic resonance imaging apparatus (1) and/or the determination of the imaging parameters is performed depending on a comparison of the imaging quality information and the corresponding reference information in the database.

2.  Method according to claim 1, **characterized in that** a pre-scan sequence or a localizer sequence is used as the second magnetic resonance sequence.

3.  Method according to claim 1 or 2, **characterized in that** a magnetic resonance sequence without excitation pulses is used as first magnetic resonance sequence.

4.  Method according to claim 3, **characterized in that** the first magnetic resonance sequence corresponds to the second magnetic resonance sequence without excitation pulses.

5.  Method according to one of the preceding claims, **characterized in that** a reference information comprising a reference signal-to-noise ratio is used, wherein the reference signal-to-noise ratio is calculated and/or empirically determined and/or determined based on previously measured landmark-specific signal-to-noise ratios stored in the database, in particular as a mean or a weighted average of the previously measured landmark-specific signal-to-noise ratios.

6.  Method according to one of the preceding claims, **characterized in that** the landmark-specific signal-to-noise ratio is determined by averaging a signal intensity within a defined image region comprising or comprised by the landmark.

7.  Method according to one of the preceding claims, **characterized in that** the imaging quality information comprises an average of the current landmark-specific signal-to-noise ratio and previously measured landmark-specific signal-to-noise ratios stored in the database, in particular as a mean and/or a sliding-window average and/or a weighted average of the previously measured landmark-specific signal-to-noise ratios.

8.  Method according to one of the preceding claims, **characterized in that** a notification to an operator of the magnetic resonance imaging apparatus (1) is generated if the performance of the magnetic resonance imaging apparatus (1) assessed from the imaging quality information meets at least one notification criterion.

9. Method according to one of the preceding claims, **characterized in that** a contrast-to-noise ratio for a pair of landmarks is determined as imaging quality information, wherein the reference information comprises a reference contrast-to-noise ratio.

10. Method according to one of the preceding claims, **characterized in that** an optimization of at least one imaging parameter of at least one imaging sequence of the current examination process is performed based on a contrast-to-noise ratio for a pair of landmarks.

11. Method according to claim 10, **characterized in that**, for the optimization of the imaging parameters, at least one modified imaging sequence differing from the imaging sequence in at least one imaging parameter value is used, wherein an optimization for the at least one imaging parameter value is performed based on a comparison of the contrast-to-noise ratio of the imaging sequence and a contrast-to-noise ratio determined from the at least one modified imaging sequence.

12. Method according to claim 11, **characterized in that** an optimized imaging sequence is generated by multiple usage of differently modified imaging protocols and an optimization of the contrast-to-noise ratio in dependence of the at least one imaging parameter.

13. A magnetic resonance imaging apparatus (1) comprising a control unit (7) configured to perform a method according to one of the preceding claims.

14. A computer program comprising instructions which, when the program is executed by a control unit of a magnetic resonance imaging apparatus according to claim 13 or an external computing unit, which communicates with a magnetic resonance imaging apparatus according to claim 13, cause the control unit or external computing unit to carry out a method according to one of the claims 1 to 12.

15. Electronically readable medium having stored thereon the computer program of claim 14.


**Patentansprüche**

1. Verfahren zur Bestimmung einer Bildgebungsqualitätsinformation für eine Magnetresonanzbildgebungsvorrichtung (1) in einem Untersuchungsprozess eines Patienten (4), der mindestens teilweise innerhalb eines Bildgebungsvolumens (5) der Magnetresonanzbildgebungsvorrichtung positioniert ist, wobei die Bildgebungsqualitätsinformation verwendet wird, um die Leistungsfähigkeit der Magnetresonanzbildgebungsvorrichtung zu beurteilen und/oder um Bildgebungsparametern für ein Bildgebungsprotokoll zu bestimmen, umfassend die folgenden Schritte:

- Messen einer Rauschinformation, welche die allgemeinen Rauscheigenschaften der Magnetresonanzbildgebungsvorrichtung (1) in dem Bildgebungsvolumen (5) unter Verwendung einer vorgegebenen ersten Magnetresonanzsequenz beschreibt,
- Erfassen von Patientenbilddaten in mindestens einer Bildgebungsregion des Patienten (4) unter Verwendung einer vorgegebenen zweiten Magnetresonanzsequenz, wobei eine Magnetresonanzsequenz, die von dem individuellen Patienten unabhängig ist, was bedeutet, dass keine Parameter der Sequenz für den Patienten adaptiert werden, als die zweite Magnetresonanzsequenz verwendet wird,
- Lokalisieren von mindestens einem anatomischen Merkpunkt von einer Gruppe vorgegebener anatomischer Merkpunkte in dem Patientenbild durch einen Merkpunktdetektierungsalgorithmus, wobei für jeden Merkpunkt und/oder für mindestens ein Paar von Merkpunkten eine Referenzinformation, welche die Bildgebungsqualitätsinformation betrifft, in einer Datenbank (8) bereitgestellt wird,
- Bestimmen eines merkpunktspezifischen Signal-zu-Rausch-Verhältnisses für jeden lokalisierten Merkpunkt aus den Patientenbilddaten an dem Merkpunkt und der Rauschinformation, und
- Bestimmen der Bildgebungsqualitätsinformation aus dem merkpunktspezifischen Signal-zu-Rausch-Verhältnis, wobei die Beurteilung der Leistungsfähigkeit der Magnetresonanzbildgebungsvorrichtung (1) und/oder die Bestimmung der Bildgebungsparameter in Abhängigkeit von einem Vergleich der Bildgebungsqualitätsinformation und der entsprechenden Referenzinformation in der Datenbank durchgeführt wird.

2. Verfahren nach Anspruch 1, **dadurch gekennzeichnet, dass** eine Vorscansequenz oder eine Lokalisierersequenz als zweite Magnetresonanzsequenz verwendet wird.

3. Verfahren nach Anspruch 1 oder 2, **dadurch gekennzeichnet, dass** eine Magnetresonanzsequenz ohne Anregungspulse als erste Magnetresonanzsequenz verwendet wird.

4. Verfahren nach Anspruch 3, **dadurch gekennzeichnet, dass** die erste Magnetresonanzsequenz der zweiten Magnetresonanzsequenz ohne Anregungspulse entspricht.

5. Verfahren nach einem der vorhergehenden Ansprüche, **dadurch gekennzeichnet, dass** eine Referenzinformation verwendet wird, welche ein Referenz-Signal-zu-Rausch-Verhältnis umfasst, wobei das Referenz-Signal-zu-Rausch-Verhältnis berechnet und/oder empirisch bestimmt und/oder basierend auf zuvor gemessenen merkpunktspezifischen Signal-zu-Rausch-Verhältnissen bestimmt wird, die in der Datenbank gespeichert sind, insbesondere als Mittelwert oder gewichteter Durchschnitt der zuvor gemessenen merkpunktspezifischen Signal-zu-Rausch-Verhältnisse.

6. Verfahren nach einem der vorhergehenden Ansprüche, **dadurch gekennzeichnet, dass** das merkpunktspezifische Signal-zu-Rausch-Verhältnis durch Mitteln einer Signalintensität innerhalb einer definierten Bildregion bestimmt wird, welche den Merkpunkt umfasst oder beinhaltet.

7. Verfahren nach einem der vorhergehenden Ansprüche, **dadurch gekennzeichnet, dass** die Bildgebungsqualitätsinformation einen Durchschnitt des aktuellen merkpunktspezifischen Signal-zu-Rausch-Verhältnisses und zuvor gemessener merkpunktspezifischer Signal-zu-Rausch-Verhältnisse umfasst, die in der Datenbank gespeichert sind, insbesondere als Mittelwert und/oder Durchschnitt eines gleitenden Fensters und/oder ein gewichteter Durchschnitt der zuvor gemessenen merkpunktspezifischen Signal-zu-Rausch-Verhältnisse.

8. Verfahren nach einem der vorhergehenden Ansprüche, **dadurch gekennzeichnet, dass** eine Benachrichtigung einer Bedienungsperson der Magnetresonanzbildgebungsvorrichtung (1) generiert wird, falls die Leistungsfähigkeit der Magnetresonanzbildgebungsvorrichtung (1), die aus der Bildgebungsqualitätsinformation beurteilt wurde, mindestens ein Benachrichtigungskriterium erfüllt.

9. Verfahren nach einem der vorhergehenden Ansprüche, **dadurch gekennzeichnet, dass** ein Kontrast-zu-Rausch-Verhältnis für ein Paar von Merkpunkten als Bildgebungsqualitätsinformation bestimmt wird, wobei die Referenzinformation ein Referenz-Kontrast-zu-Rausch-Verhältnis umfasst.

10. Verfahren nach einem der vorhergehenden Ansprüche, **dadurch gekennzeichnet, dass** eine Optimierung von mindestens einem Bildgebungsparameter von mindestens einer Bildgebungssequenz des aktuellen Untersuchungsprozesses basierend auf einem Kontrast-zu-Rausch-Verhältnis für ein Paar von Merkpunkten durchgeführt wird.

11. Verfahren nach Anspruch 10, **dadurch gekennzeichnet, dass** zur Optimierung der Bildgebungsparameter mindestens eine modifizierte Bildgebungssequenz verwendet wird, die sich von der Bildgebungssequenz in mindestens einem Bildgebungsparameterwert unterscheidet, wobei eine Optimierung für den mindestens einen Bildgebungsparameterwert basierend auf einem Vergleich des Kontrast-zu-Rausch-Verhältnisses der Bildgebungssequenz und eines Kontrast-zu-Rausch-Verhältnisses, das aus der mindestens einen modifizierten Bildgebungssequenz bestimmt wird, durchgeführt wird.

12. Verfahren nach Anspruch 11, **dadurch gekennzeichnet, dass** eine optimierte Bildgebungssequenz durch mehrfache Verwendung unterschiedlich modifizierter Bildgebungsprotokolle und eine Optimierung des Kontrast-zu-Rausch-Verhältnisses in Abhängigkeit von dem mindestens einen Bildgebungsparameter durchgeführt wird.

13. Magnetresonanzbildgebungsvorrichtung (1), umfassend eine Steuereinheit (7), die ausgelegt ist, um ein Verfahren gemäß einem der vorhergehenden Ansprüche durchzuführen.

14. Computerprogramm, umfassend Anweisungen, die bei Ausführung des Programms durch eine Steuereinheit einer Magnetresonanzbildgebungsvorrichtung nach Anspruch 13 oder eine externe Recheneinheit, die mit einer Magnetresonanzbildgebungsvorrichtung nach Anspruch 13 kommuniziert, bewirkt, dass die Steuerung oder externe Recheneinheit ein Verfahren gemäß einem der Ansprüche 1 bis 12 durchführt.

15. Elektronisch lesbares Medium, auf dem das Computerprogramm gemäß Anspruch 14 gespeichert ist.

**EP 3 502 727 B1**

**Revendications**

1. Procédé de détermination d'une information de qualité d'imagerie pour une installation (1) d'imagerie par résonance magnétique dans une opération d'examen d'un patient (4) placé au moins en partie dans un volume (5) d'imagerie de l'installation d'imagerie par résonance magnétique, dans lequel on utilise l'information de qualité d'imagerie pour évaluer la performance de l'installation d'imagerie par résonance magnétique et/ou pour déterminer des paramètres d'imagerie d'un protocole d'imagerie, comprenant les stades suivants :

   - on mesure une information de bruit décrivant les propriétés générales de bruit de l'installation (1) d'imagerie par résonance magnétique dans le volume (5) d'imagerie en utilisant une première séquence déterminée à l'avance de résonance magnétique,
   - on acquiert des données d'image du patient dans au moins une région d'imagerie du patient (4) en utilisant une deuxième séquence déterminée à l'avance de résonance magnétique, dans lequel on utilise comme deuxième séquence de résonance magnétique une séquence de résonance magnétique, qui est indépendante du patient individuel, ce qui signifie qu'aucun paramètre de la séquence n'est adapté au patient,
   - on localise au moins un repère anatomique d'un groupe de repères anatomiques déterminés à l'avance dans l'image du patient par un algorithme de détection de repère, dans lequel pour chaque repère et/ou pour au moins une paire de repères, une information de référence concernant l'information de qualité d'imagerie est prévue dans une base (8) de données,
   - on détermine un rapport signal spécifique au repère à bruit pour chaque repère localisé à partir de la donnée d'image du patient au repère et de l'information de bruit, et
   - on détermine l'information de qualité d'imagerie à partir du rapport signal spécifique au repère à bruit, dans lequel on effectue l'évaluation de la performance de l'installation (1) d'imagerie par résonance magnétique et/ou la détermination de paramètres d'imagerie en fonction d'une comparaison de l'information de la qualité d'imagerie et de l'information de référence correspondante dans la base de données.

2. Procédé suivant la revendication 1, **caractérisé en ce que** l'on utilise une séquence de pré-balayage ou une séquence de repérage comme deuxième séquence de résonance magnétique.

3. Procédé suivant la revendication 1 ou 2, **caractérisé en ce que** l'on utilise une séquence de résonance magnétique sans impulsion d'excitation comme première séquence de résonance magnétique.

4. Procédé suivant la revendication 3, **caractérisé en ce que** la première séquence de résonance magnétique correspond à la deuxième séquence de résonance magnétique sans impulsion d'excitation.

5. Procédé suivant l'une des revendications précédentes, **caractérisé en ce que** l'on utilise une information de référence comprenant un rapport signal de référence à bruit, dans lequel on calcule le rapport signal de référence à bruit et/ou on le détermine empiriquement et/ou on le détermine sur la base de rapports signal spécifique à un repère à bruit mesurés précédemment et mis en mémoire dans la base de données, en particulier sous la forme d'une moyenne ou d'une moyenne pondérée des rapports mesurés précédemment de signal spécifique à un repère à bruit.

6. Procédé suivant l'une des revendications précédentes, **caractérisé en ce que** l'on détermine le rapport signal à bruit spécifique à un repère en faisant la moyenne d'une intensité du signal dans une région d'image définie comprenant ou comprise par le repère.

7. Procédé suivant l'une des revendications précédentes, **caractérisé en ce que** l'information de qualité d'imagerie comprend une moyenne du rapport en cours signal spécifique à un repère à bruit et des rapports mesurés précédemment de signal spécifique à un repère à bruit mis en mémoire dans la base de données, en particulier sous la forme d'une moyenne ou d'une moyenne mobile et/ou d'une moyenne pondérée des rapports mesurés précédemment de signal à bruit spécifique à un repère.

8. Procédé suivant l'une des revendications précédentes, **caractérisé en ce que** l'on produit une notification à un opérateur de l'installation (1) d'imagerie par résonance magnétique si la performance de l'installation (1) d'imagerie par résonance magnétique évaluée à partir de l'information de qualité d'imagerie, satisfait au moins un critère de notification.

9. Procédé suivant l'une des revendications précédentes, **caractérisé en ce que** l'on détermine un rapport de contraste

13

à bruit pour une paire de repères comme information de qualité d'imagerie, l'information de référence comprenant un rapport de contraste à bruit de référence.

10. Procédé suivant l'une des revendications précédentes, **caractérisé en ce que** l'on effectue une optimisation d'au moins un paramètre d'imagerie d'au moins une séquence d'imagerie de l'opération d'examen en cours sur la base d'un rapport de contraste à bruit pour une paire de repères.

11. Procédé suivant la revendication 10, **caractérisé en ce que**, pour l'optimisation des paramètres d'imagerie, on utilise au moins une séquence d'imagerie modifiée différente de la séquence d'imagerie par au moins une valeur de paramètre d'imagerie, dans lequel on effectue une optimisation de la au moins une valeur de paramètre d'imagerie sur la base d'une comparaison du rapport de contraste à bruit de la séquence d'imagerie et d'un rapport de contraste à bruit déterminé à partir de la au moins une séquence d'imagerie modifiée.

12. Procédé suivant la revendication 11, **caractérisé en ce que** l'on produit une séquence d'imagerie optimisée par utilisation multiple de protocoles d'imagerie modifiés de manière différente et par une optimisation du rapport contraste à bruit en fonction du au moins un paramètre d'imagerie.

13. Installation (1) d'imagerie par résonnance magnétique, comprenant une unité (7) de commande configurée pour effectuer un procédé suivant l'une des revendications précédentes.

14. Programme d'ordinateur comprenant des instructions qui, lorsque le programme est exécuté par l'unité de commande d'une installation d'imagerie par résonnance magnétique suivant la revendication 13 ou par une unité informatique extérieure, qui communique avec une installation d'imagerie par résonnance magnétique suivant la revendication 13, fait que l'unité de commande et/ou l'unité informatique extérieure effectue un procédé suivant l'une des revendications 1 à 12.

15. Support déchiffrable électroniquement sur lequel est mémorisé le programme d'ordinateur de la revendication 14.

# FIG 1

# FIG 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120010495 A1

- US 2017156630 A1

**Non-patent literature cited in the description**

- Obese patients in an open MRI at 1.0 Tesla: image quality, diagnostic impact and feasibility. **MAXIMIL-IAN DE BUCOURT et al.** EUROPEAN RADIOLOGY. SPRINGER, 03 December 2010, vol. 21

- **X. S. ZHOU ; Z. PENG ; Y. ZHAN ; M. DEWAN ; B. JIAN ; A. KRISHNAN ; Y. TAO ; M. HARDER ; S. GROSS-KOPF ; U. FEUERLEIN.** Redundancy, redundancy, redundancy: the three keys to highly robust anatomical parsing in medical images. In MIR '10: Proc. *Int'l Conf. Multimedia Info Retrieval,* 2010, 175-184